# EUROPÄISCHE PATENTSCHRIFT

(11) **EP 1 015 411 B1**
(45) Veröffentlichungstag und Bekanntmachung des Hinweises auf die Patenterteilung: **11.06.2003**
(21) Anmeldenummer: 98950022.8
(22) Anmeldetag: 10.09.1998
(51) Int. Cl.: C07C 57/07

(54) **VERFAHREN ZUR FRAKTIONIERTEN KONDENSATION EINES ACRYLSÄURE ODER METHACRYLSÄURE ENTHALTENDEN HEISSEN GASGEMISCHES MIT EINEM HOHEN ANTEIL NICHT KONDENSIERBARER KOMPONENTEN**
METHOD FOR THE FRACTIONAL CONDENSATION OF A HOT GAS MIXTURE CONTAINING ACRYLIC ACID OR METHACRYLIC ACID AND HAVING A HIGH PROPORTION OF NON-CONDENSABLE CONSTITUENTS
PROCEDE POUR LA CONDENSATION FRACTIONNEE D'UN MELANGE GAZEUX CHAUD CONTENANT UN ACIDE ACRYLIQUE OU UN ACIDE METHACRYLIQUE ET PRESENTANT UNE PROPORTION ELEVEE DE CONSTITUANTS NON CONDENSABLES

(30) Priorität: 12.09.1997 DE 19740253
(43) Veröffentlichungstag der Anmeldung: 05.07.2000
(73) Patentinhaber: BASF AKTIENGESELLSCHAFT, 67056 Ludwigshafen (DE)
(72) Erfinder: ECK, Bernd, D-68519 Viernheim (DE); MACHHAMMER, Otto, D-68163 Mannheim (DE); PROLL, Theo, D-67098 Bad Dürkheim (DE); SCHLIEPHAKE, Volker, D-67105 Schifferstadt (DE); THIEL, Joachim, D-67433 Neustadt (DE); BRÖLLOS, Klaus, D-64342 Seeheim-Jugenheim (DE)
(74) Vertreter: Isenbruck, Günter, Dr.
(86) Internationale Anmeldenummer: EP9805779
(87) Internationale Veröffentlichungsnummer: WO99014182

(56) Entgegenhaltungen:
- EP-A- 0 398 226
- EP-A- 0 751 110
- US-A- 4 199 410

## Beschreibung

Die vorliegende Erfindung betrifft ein Verfahren zur fraktionierten Kondensation eines heißen Gasgemisches, das wenigstens zwei kondensierbare Komponenten und einen hohen Anteil nicht kondensierbarer Komponenten enthält.

Heiße Gasgemische, die neben kondensierbaren Komponenten einen hohen Anteil nicht kondensierbarer Komponenten enthalten, entstehen z.B. bei der Herstellung von Acrylsäure oder Methacrylsäure durch heterogen katalysierte Gasphasenoxidation. Hierbei wird zum Beispiel Propen mit molekularem Sauerstoff an im festen Aggregatzustand befindlichen Katalysatoren bei Temperaturen zwischen 200 und 400 °C einstufig oder zweistufig über Acrolein umgesetzt (vgl. z.B. DE-A-1 962 431, DE-A-2 943 707, DE-C-1 205 502, EP-A-0 257 565, EP-A-0 253 409, DE-A-2 251 364, EP-A-0 117 146, GB-B-1 450 986 und EP-A-0 293 224). Es werden oxidische Mehrkomponenten-Katalysatoren z.B. auf der Basis von Oxiden der Elemente Molybdän, Bismut und Eisen (in der I. Stufe) bzw. Molybdän und Vanadium (in der II. Stufe) eingesetzt. Das entstehende heiße Reaktionsgasgemisch enthält neben der (kondensierbaren) Acrylsäure oder der (kondensierbaren) Methacrylsäure und kondensierbaren Nebenkomponenten einen hohen Anteil nicht kondensierbarer Komponenten wie Stickstoff oder Sauerstoff.

Zur Abtrennung der Acrylsäure sind zahlreiche Verfahren bekannt. So ist aus DE-C-2 136 396 bekannt, die Acrylsäure aus den bei der katalytischen Gasphasenoxidation erhaltenen Reaktionsgasen durch Gegenstromabsorption mit einem Gemisch aus etwa 75 Gew.-% Diphenylether und etwa 25 Gew.-% Diphenyl abzutrennen. DE-A-2 449 780 beschreibt das Abkühlen des heißen Reaktionsgases durch Teilverdampfen des Lösungsmittels in einem Direktkondensator (Quenchapparat) vor der Gegenstromabsorption. Neben dieser Absorption des die Acrylsäure enthaltenen Reaktionsprodukts in ein hochsiedendes Lösungsmittelgemisch sehen andere Verfahren eine Totalkondensation von Acrylsäure und des weiterhin bei der katalytischen Oxidation entstehenden Reaktionswassers vor. Dabei entsteht eine wäßrige Acrylsäurelösung, die über Destillation mit einem Azeotropmittel (vgl. z.B. DE-C-3 429 391 und JP-A-1 124 766) oder über ein Extraktionsverfahren (vgl. z.B. DE-A-2 164 767 und JP-A-5 81 40-039) weiter aufgearbeitet werden kann. In EP-A-0 551 111 wird das mittels Gasphasenoxidation hergestellte, acrylsäurehaltige Gemisch mit Wasser in einem Absorptionsturm in Berührung gebracht und die erhaltene wäßrige Lösung in Anwesenheit eines Lösungsmittels, das mit polaren Leichtsiedern wie Wasser oder Essigsäure ein Azeotrop bildet, destilliert. Daneben ist aus DE-C-2 323 328 die Abtrennung von Acrylsäure aus einer wäßrigen Butanol-Acrylsäure-Veresterungsablauge durch Extraktion mit einem speziellen Gemisch organischer Lösungsmittel bekannt. Nachteilig bei den hier beschriebenen Verfahren ist, daß zur Extraktion oder Absorption ein organisches Lösungsmittel verwendet wird, das in einer weiteren Verfahrensstufe, wie eine Rektifikation, bei hoher thermischer Belastung wieder abgetrennt werden muß.

EP-A-0 751 110 offenbart ein zweistufiges Verfahren zur Reinigung von Acrolein, das in einem Ausgangsgasstrom neben Wasser, Säuren und inerten Gasen enthalten ist. In der ersten Stufe wird das Ausgangsgas in einer Kühlkolonne in einen flüssigen Strom und einen gasförmige Austrag getrennt, wobei die Kühlkolonne unter definierten Bedingungen betrieben wird. In der zweiten Stufe wird der gasförmige Austrag bei einer um 20 °C niedrigeren Temperatur unter Erhalt einer flüssigen Fraktion und einer gereinigten gasförmigen Fraktion kondensiert.

Die Aufgabe der vorliegenden Erfindung bestand darin, ein Verfahren hervorzubringen, das ein bei der katalytischen Gasphasenoxidation zu Acrylsäure oder Methacrylsäure entstehendes Gasgemisch, das einen hohen Anteil an nicht kondensierbaren Komponenten aufweist, so aufzutrennt, daß Acrylsäure oder Methacrylsäure in möglichst hoher Reinheit bei Benötigung möglichst weniger Verfahrensstufen erhalten wird.

Die Lösung dieser Aufgabe geht aus von dem Verfahren zur fraktionierten Kondensation eines Gasgemisches, das neben Acrylsäure oder Methacrylsäure noch mindestens eine weitere kondensierbare Komponente und außerdem einen hohen Anteil einer oder mehrerer nicht kondensierbarer Komponenten enthält. Das erfindungsgemäße Verfahren zur fraktionierten Kondensation ist dadurch gekennzeichnet, daß man das Gasgemisch durch eine Kolonne mit trennwirksamen Einbauten führt und die kondensierbaren Komponenten durch Kühlung auskondensiert.

Erfindungsgemäß wird auch ein Verfahren zur Herstellung von Acrylsäure oder Methacrylsäure bereitgestellt. Dieses geht aus von der katalytischen Gasphasenoxidation von C₃-/C₄-Alkanen, -Alkenen, -Alkanolen und/oder -Alkanalen und/oder Vorstufen davon unter Entstehung eines Rohproduktes, das neben Acrylsäure oder Methacrylsäure noch mindestens ein Nebenprodukt und/oder mindestens einen nichtumgesetzten Ausgangsstoff enthält.
Das erfindungsgemäße Herstellungsverfahren ist dadurch gekennzeichnet, daß das gasförmige Rohprodukt nach dem vorstehenden erfindungsgemäßen Verfahren zur fraktionierten Kondensation aufgearbeitet wird.

In einer bevorzugten Ausführungsform enthält das heiße, die Acrylsäure oder Methacrylsäure enthaltende Gasgemisch eine Schwersieder-, Mittelsieder- und Leichtsiederfraktion, von denen jede wiederum eine oder mehrere Komponenten enthält, wie es z.B. bei dem bei der Acrylsäureherstellung durch katalytische Gasphasenoxidation entstehenden heißen Reaktionsproduktgasgemisch der Fall ist. Weitere bevorzugte Ausführungsformen der Erfindung ergeben sich aus der nachfolgenden Beschreibung und dem Beispiel. Die Verwendung einer Kolonne mit trennwirksamen Einbauten zur fraktionierten Kondensation von heißen Gasgemischen mit einem hohen Anteil an nicht kondensierbaren Komponenten wird beschrieben.

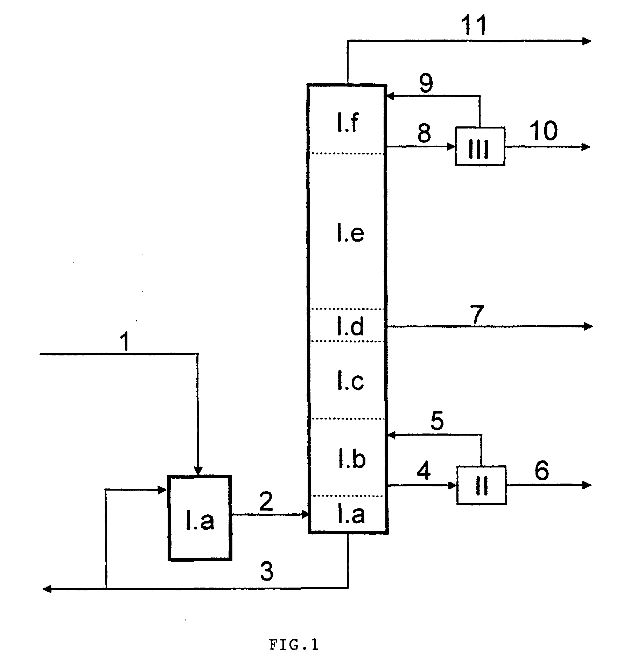

Die einzige Figur zeigt schematisch eine Kolonne, die bevorzugt zur Durchführung des erfindungsgemäßen Verfahrens eingesetzt wird.

Die für das erfindungsgemäße Verfahren einsetzbaren Kolonnen unterliegen keiner besonderen Beschränkung. Grundsätzlich eignen sich alle Kolonnen mit trennwirksamen Einbauten. Als Kolonneneinbauten kommen alle gängigen Einbauten in Betracht, insbesondere Böden, Packungen und/oder Füllkörper. Von den Böden sind Glockenböden, Siebböden, Ventilböden und/oder Dual-Flow-Böden bevorzugt. Die Kolonne umfaßt wenigstens eine Kühlvorrichtung. Hierfür eignen sich alle Wärmeübertrager oder Wärmetauscher, bei denen die bei der Kondensation frei werdende Wärme indirekt (extern) abgeführt wird. Hierfür können alle gängigen Apparate eingesetzt werden, wobei Rohrbündelwärmetauscher, Plattenwärmetauscher und Lufkühler bevorzugt sind. Geeignete Kühlmedien sind bei einem Luftkühler entsprechend Luft und bei anderen Kühlvorrichtungen Kühlflüssigkeiten, insbesondere Wasser. Ist nur eine Kühlvorrichtung vorgesehen, so wird diese am Kopf der Kolonne eingebaut, in dem die Leichtsiederfraktion auskondensiert wird. Der Fachmann kann in Abhängigkeit von der gewünschten Reinheit der kondensierten Fraktionen und damit der Komponenten die Anzahl der erforderlichen Kühlvorrichtungen leicht bestimmen, wobei die Reinheit der kondensierten Komponenten im wesentlichen durch die installierte Trennleistung der Kolonne, d.h. die Kolonnenhöhe und die über das zu kondensierende Gasgemisch eingetragene Energie bestimmt wird. Zweckmäßigerweise werden bei Vorhandensein mehrerer Kühlvorrichtungen diese in verschiedenen Abschnitten der Kolonne eingebaut. Z.B. können bei einem heißen Gasgemisch, das neben dem hohen Anteil nicht kondensierbarer Komponenten eine Schwersieder-, Mittelsieder- und Leichtsiederfraktion enthält, eine Kühlvorrichtung im unteren Abschnitt der Kolonne zur Auskondensation der Schwersiederfraktion und eine Kühlvorrichtung am Kopf der Kolonne zur Auskondensation der Leichtsiederfraktion vorgesehen sein. Die kondensierten Fraktionen werden an den jeweiligen Abschnitten in der Kolonne über Seitenabzüge abgeführt. In Abhängigkeit von der Anzahl der Komponenten in der Schwersieder-, Mittelsieder- und Leichtsiederfraktion können jeweils auch mehrere Seitenabzüge vorgesehen sein. Die über die Seitenabzüge abgezogenen Fraktionen können dann weiteren Reinigungsstufen unterzogen werden, z.B. destillativen oder extraktiven Trennverfahren oder einer Kristallisation, je nach gewünschter Reinheit der Komponenten. In einer bevorzugten Ausgestaltung der Erfindung sind ein Schwersiederabzug, ein Leichtsiederabzug und 0, 1 oder 2 Mittelsiederabzüge vorgesehen. Der in der Kolonne vorliegende Druck hängt von der Menge an nicht kondensierbaren Komponenten ab und beträgt vorzugsweise 0,5 - 5 bar Absolutdruck, insbesondere 0,8 - 3 bar Absolutdruck. Die genauen Betriebsbedingungen für die Kolonne, wie Temperaturund Druckführung, Schaltung und Anordnung der Kühlvorrichtung(en), Anordnung der Seitenabzüge zum Abziehen der gewünschten Fraktionen, Wahl der Kolonnenhöhe und des Kolonnendurchmessers, Anzahl und Abstand der trennwirksamen Einbauten/Böden in der Kolonne oder Art der trennwirksamen Kolonneneinbauten, können vom Fachmann im Rahmen fachüblicher Versuche in Abhängigkeit von der Trennaufgabe ermittelt werden.

Als Gasgemische sind alle heißen Gasgemische einsetzbar, die neben Acrylsäure oder Methacrylsäure wenigstens eine weitere kondensierbare Komponente und einen hohen Anteil wenigstens einer nicht kondensierbaren Komponente enthalten und keine ausgeprägte Azeotropbildung zeigen. D.h. Azeotrope sind dann noch geeignet, wenn die Zusammensetzung des Azeotropengemisches sehr weit nach einer Seite verschoben ist. Erfindungsgemäß fallen unter den Begriff "kondensierbare Komponente" alle Komponenten oder Verbindungen, deren Siedepunkt nicht niedriger als - 40 °C bei Normaldruck (1 bar) liegt, vorzugsweise nicht niedriger als - 30 °C bei Normaldruck (1 bar), insbesondere nicht niedriger als - 20 °C bei Normaldruck (1 bar). Vorteilhafterweise liegt die Temperatur des heißen, zu kondensierenden Gasgemisches zwischen 20 und 450 °C, insbesondere zwischen 100 und 350 °C und am meisten bevorzugt zwischen 150 und 300°C. Der Anteil an nicht kondensierbarer/en Komponente(n) liegt vorteilhafterweise zwischen 20 und 100 Gew.-%, insbesondere zwischen 50 und 95 Gew.-%, am meisten bevorzugt zwischen 70 und 90 Gew.-%, jeweils bezogen auf 100 Gew.-% heißes Gasgemisch. Das Gasgemisch enthält in einer bevorzugten Ausgestaltung der Erfindung eine Schwersieder-, Mittelsieder- und Leichtsiederfraktion, von denen jede wiederum eine oder mehrere Komponenten enthalten kann, wobei die Acrylsäure oder Methacrylsäure in der Mittelsiederfraktion erscheint. Die Begriffe Schwersieder- und Leichtsiederfraktionen sind vorliegend auf das gewünschte Produkt, die Acrylsäure oder Methacrylsäure, die in der Mittelsiederfraktion erscheint, bezogen und bezeichnen die Fraktionen, die einen niedrigeren bzw. höheren Siedepunkt bzw. Siedebereich haben als die Mittelsiederfraktion, wobei diese um wenigstens 5 bis 10 C° vom Siedebereich/-punkt der Mittelsiederfraktion abweichen.

In einer bevorzugten Ausführungsform wird das heiße Gasgemisch vor der Auskondensation direkt oder indirekt (extern) abgekühlt. Dies kann über indirekte Kühlung, z.B. Gaskühler, aber auch über direkte Kühlung mit einem kaltenden schwersiedenden Hilfsstoff, z.B. hochsiedenden Kohlenwasserstoffen, oder bevorzugter Weise mit der aus dem Gasgemisch kondensierten Schwersiederfraktion erfolgen. Bei dem Hilfsstoff ist jedoch nachteilig, daß dieser wieder aufgearbeitet werden muß. Apparativ kann die Abkühlung getrennt von der Kolonne in einem eigenen Apparat, z.B. einem Gaskühler, einem Quench oder einem Flashtopf, oder im Sumpfbereich der Kolonne integriert (mit oder ohne Kolonneneinbauten) erfolgen. Bei der Abkühlung wird das heiße Gasgemisch auf 50 bis zu 300 C°, insbesondere 70 bis 200 C°, jeweils unterhalb des Siedepunkts der schwerstsiedenden Komponente abgekühlt.

Ein besonders geeignetes heißes Gasgemisch ist das Reaktionsgasgemisch, wie es bei der katalytischen Gasphasenoxidation von C₃- bzw. C₄-Alkanen, -Alkenen, -Alkanolen und/oder -Alkanalen und/oder Vorstufen davon zu Acrylsäure bzw. Methacrylsäure nach bekannten Verfahren entsteht. Besonders vorteilhaft wird Propen, Propan, Acrolein, tert.-Butanol, Isobuten, Isobutan, Isobutyraldehyd, Methacrolein, Isobuttersäure oder Methyl-tert.butylether eingesetzt. Als Ausgangsverbindungen sind aber auch solche verwendbar, aus denen sich die eigentliche C₃-/ C₄- Ausgangsverbindung erst während der Gasphasenoxidation intermediär bildet. Beispielhaft genannt seien für die Herstellung der Methacrylsäure Isobuttersäure oder Methyl-tert.butylether. Als Rohprodukt für die Kondensation liegt somit vorzugsweise ein Gasgemisch der katalytischen Gasphasenoxidation von C₃-/C₄-Alkanen, -Alkenen, -Alkanolen und/oder -Alkanalen und/oder Vorstufen davon zu Acrylsäure oder zu Methacrylsäure vor. Sowohl Acrylsäure als auch Methacrylsäure können direkt aus Propan bzw. Isobutan hergestellt werden. Bei Einsatz von Propan als Ausgangsstoff kann dieses nach bekannten Verfahren durch katalytische Oxidehydrierung (z.B. nach US-A-5 510 558), homogene Oxidehydrierung (z.B. nach CN-A-1 105 352) oder katalytische Dehydrierung (z.B. nach EP-A-0 253 409) zu einem Propen-/Propan-Gemisch umgesetzt werden. Geeignete Propen-/Propan-Gemische sind auch Raffineriepropen (70 % Propen und 30 % Propan) oder Crackerpropen (95 % Propen und 5 % Propan). Bei Einsatz eines Propen-/Propan-Gemisches zur Herstellung von Acrylsäure wirkt Propan als Verdünnungsgas und/oder Reaktant. Ebenso wie Propan kann auch Isobutan als Reaktand wirken und beide können z.B. gemäß EP-B-0 608 838 direkt zu Acrylsäure bzw. Methacrylsäure umgesetzt werden. Bei der Herstellung der Acrylsäure bzw. Methacrylsäure werden in der Regel die Ausgangsgase mit inerten Gasen wie Stickstoff, CO₂, gesättigten C₁-C₆-Kohlenwasserstoffen und/oder Wasserdampf verdünnt, im Gemisch mit Sauerstoff bei erhöhten Temperaturen (üblicherweise 200 bis 450 °C) sowie gegebenenfalls erhöhtem Druck über übergangsmetallische (z.B. Mo, V, W und/oder Fe enthaltende) Mischoxidkatalysatoren geleitet und oxidativ in die Acrylsäure bzw. Methacrylsäure umgewandelt (vgl. z.B. DE-A-4 405 059, EP-A-0 253 409, EP-A-0 092 097 und DE-A-4 431 949). Diese Umsetzungen werden beispielsweise einstufig oder mehrstufig durchgeführt. Besonders geeignete Verfahren zur Herstellung von Methacrylsäure sind solche, die von Methacrolein ausgehen, insbesondere wenn das Methacrolein durch gasphasenkatalytische Oxidation von tert.-Butanol, Isobutan oder Isobuten oder durch Umsetzung von Formaldehyd mit Propionaldehyd gemäß EP-B-0 092 097 und EP-B-0 058 927 erzeugt wird. Das entstehende Reaktionsgasgemisch enthält neben der gewünschten Säure Nebenkomponenten wie nicht umgesetztes Acrolein bzw. Methacrolein und/oder Propen bzw. Isobuten, Wasserdampf, Kohlenmonoxid, Kohlendioxid, Stickstoff, Sauerstoff, Essigsäure, Propionsäure, Formaldehyd, weitere Aldehyde und Maleinsäureanhydrid. Üblicherweise enthält das Reaktionsgasgemisch, jeweils bezogen auf das gesamte Reaktionsgasgemisch, 1 bis 30 Gew.-% Acrylsäure bzw. Methacrylsäure, 0,05 bis 1 Gew.-% Propen bzw. Isobuten und 0,05 bis 1 Gew.-% Acrolein bzw. Methacrolein, 0,05 bis 10 Gew.-% Sauerstoff, 0,05 bis 2 Gew.-% Essigsäure, 0,01 bis 2 Gew.-% Propionsäure, 0,05 bis 1 Gew.-% Formaldehyd, 0,05 bis 2 Gew.-% Aldehyde, 0,01 bis 0,5 Gew.-% Maleinsäureanhydrid und 20 bis 98 Gew.-%, vorzugsweise 50 - 90 Gew.-% inerte Verdünnungsgase. Als inerte Verdünnungsgase sind insbesondere gesättigte C₁ - C₆-Kohlenwasserstoffe, wie 0 bis 90 Gew.-% Methan und/oder Propan, daneben 1 bis 30 Gew.-% Wasserdampf, 0,05 bis 15 Gew.-% Kohlenoxide und 0 bis 90 Gew.-% Stickstoff, jeweils bezogen auf 100 Gew.-% Verdünnungsgas, enthalten. Somit enthält ein solches Gasgemisch neben der Zielkomponente Acrylsäure bzw. Methacrylsäure, die als Mittelsiederfraktion kondensiert, weitere Verbindungen im Schwersieder- und Leichtsiederbereich. Zweckmäßigerweise wird die fraktionierte Kondensation dann dergestalt durchgeführt, daß in der Kolonne zwei Kühlvorrichtungen vorgesehen sind, eine im unteren Bereich der Kolonne zur Auskondensation der schwersiedenden Komponenten und eine im oberen Bereich der Kolonne zur Auskondensation der Leichtsiederfraktion.

Vorteilhafterweise wird das Verfahren bei Vorhandensein einer Schwersiederfraktion, einer Mittelsiederfraktion, einer Leichtsiederfraktion und nicht kondensierbarer Komponente(n) so durchgeführt wie es in der Figur gezeigt ist und wie es im folgenden beschrieben ist, wobei sich die Kolonne in verschiedene Abschnitte untergliedern läßt, in denen unterschiedliche verfahrenstechnische Aufgaben gelöst werden. Die Bezugsziffern in der Figur bezeichnen hierbei die einzelnen Abschnitte in der Kolonne (I.a bis I.f) bzw. separate Abschnitte/Apparate von der Kolonne (I.a), Zu- und Ableitungen (1 - 11) sowie die Kühlkreise II und III.

### I.a Sumpfbereich:

### Abkühlung des heißen Gasgemisches

Im Sumpfbereich I.a wird das heiße Gasgemisch eingeleitet und abgekühlt. Dies kann über indirekte Kühlung, z.B. Wärmetauscher, oder direkte Kühlung mit im nächsten Abschnitt der Kolonne kondensierter Schwersiederfraktion als Kühlmedium erfolgen. Das Abkühlen kann statt im Sumpfbereich der Kolonne auch analog getrennt von der Kolonne in einem separaten Apparat I.a erfolgen, wie es in der Figur gezeigt ist. In diesem Fall wird das heiße, zu kondensierende Gasgemisch aus Leitung 1 in einem Quench bzw. Vorquench I.a abgekühlt und über Leitung 2 dem Sumpfbereich I.a der Kolonne zugeführt. Über Leitung 3 wird das Kühlmedium (kondensierte Schwersiederfraktion) zur Kühlung des heißen Gasgemisches in den Quench bzw. Vorquench zurückgeführt.

Wird die Kühlung mit einem schwersiedenden Hilfsstoff bzw. mit der Schwersiederfraktion aus Bereich I.b. durchgeführt, kann ein Teil des Stromes, üblicherweise weniger als 1 Gew.-% bezogen auf 100 Gew.-% Kondensat im Seitenabzug, aus dem Prozeß ausgeschleust werden.

### I.b Kühlkreis II:

### Kondensation der Schwersiederfraktion

Im Kolonnenabschnitt I.b wird die Kondensationswärme extern über Kühlkreis II mittels eines Wärmetauschers mit z.B. Wasser als Kühlmedium abgeführt, indem kondensierte Schwersiederfraktion über Leitung 4 aus der Kolonne abgeführt wird, gekühlt wird und ein Teil der gekühlten, kondensierten Schwersiederfraktion über Leitung 5 der Kolonne rückgeführt wird, während der andere Teil, üblicherweise weniger als 1 Gew.-% bezogen auf 100 Gew.-% Kondensat im Seitenabzug, über Leitung 6 ausgeschleust wird. Die rückgeführte, kondensierte Schwersiederfraktion wird im Gegenstrom zum aufsteigenden Gas geführt. In Abhängigkeit von der Trennaufgabe besteht auch die Möglichkeit, die Abschnitte I.a und I.b der Kolonne, d.h. die Abkühlung des Reaktionsgases und die Kondensation einer Schwersiederfraktion, apparativ zu vereinigen (nicht gezeigt), so daß die genannten Vorgänge gleichzeitig durchgeführt werden. Es besteht auch die Möglichkeit statt des externen Kühlkreises II eine direkte Kühlung vorzusehen (nicht gezeigt), bei der ein schwersiedender Hilfsstoff zur Abkühlung eingespritzt wird, der wiederum im Kreis geführt oder extern aufgearbeitet wird.

### I.c Kühlkreis II → Seitenabzug:

### Schwersiederanreichung

Im Kolonnenabschnitt I.c zwischen Kolonnenabschnitt I.b (Kühlkreis II) und I.d (Seitenabzug) erfolgt zum Kühlkreis II hin eine destillative Anreicherung und Auskondensation der Schwersiederfraktion aus dem im Gegenstrom nach oben geführten Gasstrom.

### I.d Seitenabzug:

### Abziehen der Mittelsiederfraktion

Über Seitenabzug 7 im Kolonnenabschnitt I.d. werden gewünschte Zielkomponenten wie Acrylsäure bzw. Methacrylsäure abgeführt. Im Grenzfall einer einstufigen Kondensation wird im Bereich des Seitenabzugs 7 die Mittelsiederfraktion aus dem im Gegenstrom nach oben geführten Gasgemisch auskondensiert.

### I.e Seitenabzug → Kühlkreis III:

### Mittelsiederanreicherung

Im Kolonnenabschnitt I.e zwischen Kolonnenabschnitt I.d (Seitenabzug 7) und I.f (Kühlkreis III) erfolgt die destillative Anreicherung der Mittelsiederfraktion aus dem im Gasgemisch nach oben geführten Gasstrom, wobei die Mittelsiederfraktion zum Seitenabzug (Bereich I.d) hin angereichert wird. Es besteht auch die Möglichkeit, apparativ die Abschnitte I.d und I.e der Kolonne zu einem Abschnitt zusammenzufassen (nicht gezeigt). Zweckmäßigerweise wird in diesem Fall in Abschnitt I.d der Kolonne ein Fangboden eingebaut, um die Flüssigkeit aus der Kolonne abzuziehen.

### I.f Kühlkreis III:

### Kondensation der Leichtsiederfraktion

Im Kolonnenabschnitt I.f des externen Kühlkreises III erfolgt die Kondensation der Leichtsiederfraktion aus dem im Gegenstrom nach oben geführten Gasstrom. Analog zu Kühlkreis II wird die Kondensationswärme extern über Kühlkreis III mittels eines Wärmetauschers mit z.B. Wasser als Kühlmedium abgeführt, indem kondensierte Leichtsiederfraktion über Leitung 8 abgezogen wird, gekühlt wird und ein Teil der gekühlten, kondensierten Leichtsiederfraktion über Leitung 9 der Kolonne rückgeführt wird, während der andere Teil über Leitung 10 ausgeschleust wird. Die nicht kondensierten Gase werden vom Kopf der Kolonne über Leitung 11 abgezogen, wobei ggf. der Gasstrom noch überhitzt werden kann, um eine weitere Kondensation im Brüdenrohr zu vermeiden.

Die in der Figur schematisch gezeigte Kolonne eignet sich besonders gut zur fraktionierten Kondensation eines oben genannten Reaktionsgasgemisches wie es bei der katalytischen Gasphasenoxidation zu Acrylsäure bzw. Methacrylsäure entsteht. In diesem Fall wird zur Vermeidung einer Polymerisation vorteilhafterweise an einer oder mehreren, vom Fachmann leicht zu ermittelnden Stellen in der Kolonne ein Stabilisator zugegeben, insbesondere Phenothiazin oder ein anderer, in EP-A-0 765 856 offenbarter Stabilisator. Von der Kolonne wird über Seitenabzug 7 das Zielprodukt, die Acrylsäure, bzw. Methacrylsäure, in einer hohen Reinheit von über 95 Gew.-%, insbesondere über 97 Gew.-%, jeweils bezogen auf das im Seitenabzug abgezogene Kondensat, abgenommen. Die nicht kondensierbaren Komponenten, bei denen es sich vorzugsweise um Stickstoff, Kohlenmonoxid, Kohlendioxid, Sauerstoff, Methan, Propan und Propen, handelt, werden vom Kopf der Kolonne über Leitung 11 abgezogen. Die im Abschnitt I.b auskondensierte Schwersiederfraktion, die überwiegend Maleinsäureanhydrid, Benzoesäure, Stabilisatoren wie Phenothiazin oder andere, in EP-A-0 765 856 offenbarte Stabilisatoren, monomere und oligomere Acrylsäure enthält, wird über Leitung 6 ausgeschleust, während die auskondensierte Leichtsiederfraktion, die überwiegend Wasser, Essigsäure und Formaldehyd enthält, über Leitung 10 abgezogen wird.

Anders als die bisherigen Verfahren zur Abtrennung von Acrylsäure, die die Verwendung eines Absorptions- oder Extraktionsmittels erfordern, ist erfindungsgemäß eine Abtrennung der Acrylsäure bzw. Methacrylsäure in hoher Reinheit dadurch möglich, daß man die heißen Reaktionsgase aus der katalytischen Gasphasenoxidation von unten in eine Absorptionskolonne führt und in sich selber aufsteigen läßt, ohne ein externes Absorptionsmittel im Gegenstrom zu führen. Die aufsteigenden Gase kühlen ab, die kondensierbaren Bestandteile kondensieren und bilden quasi ein absteigendes "internes Absorptionsmittel". Damit ermöglicht das erfindungsgemäße Verfahren die Abtrennung der Acrylsäure bzw. Methacrylsäure in hoher Reinheit ohne externes Absorptions- oder Extraktionsmittel. Weiterhin ermöglicht das Verfahren eine optimale Nutzung der Wärmeenergie, die in den heißen Reaktionsgasen aus der katalytischen Gasphasenoxidation enthalten ist. Darüberhinaus können die über Kopf abgezogenen, nicht kondensierbaren Komponenten als Verdünnungs- oder Kreisgas in die Stufe zur Herstellung der Acrylsäure bzw. Methacrylsäure zurückgeführt werden.

Das erfindungsgemäße Verfahren ermöglicht in nur einer Verfahrensstufe nicht nur eine Auftrennung eines heißen Gasgemisches in kondensierbaren Anteil und nicht kondensierbaren Anteil, sondern gleichzeitig eine Auftrennung des kondensierbaren Anteils in bei verschiedenen Siedepunkten bzw. Siedebereichen siedende Fraktionen und damit eine hohe Reinheit der verschiedenen Komponenten. Wie gesagt, bilden die kondensierten Komponenten quasi ein absteigendes "internes Absorptionsmittel", weshalb man das erfindungsgemäße Verfahren vereinfacht als vorteilhafte Kombination von Rektifikation und Absorption ansehen kann. Besonders überraschend ist, daß diese hohe Reinheit der Komponenten ohne Verwendung eines Hilfsstoffes möglich ist. In optimaler Weise nutzt das erfindungsgemäße Verfahren die Wärmeenergie von Gasgemischen aus Reaktionen, bei denen heiße Gasgemische entstehen. Von herkömmlichen Destillations-, Rektifikations- und Kondensationsverfahren unterscheidet sich das erfindungsgemäße Verfahren darin; daß bei einem hohen Anteil nicht kondensierbarer Komponenten gearbeitet wird. Üblicherweise beträgt bei den oben genannten herkömmlichen Verfahren der Anteil nicht kondensierbarer Komponenten weniger als 5 %. Damit ermöglicht das erfindungsgemäße Verfahren eine besonders wirtschaftliche Auftrennung eines heißen Gasgemisches mit einem hohen Anteil nicht kondensierbarer Komponenten in dessen kondensierbare Komponenten in einer einzigen Verfahrensstufe. Besonders vorteilhaft ist, daß die gewünschten Komponenten in hoher Reinheit erhalten werden können.

Die Erfindung wird anhand des folgenden Beispiels erläutert, das eine bevorzugte Ausführungsform der Erfindung darstellt.

### Beispiel:

Aus einer katalytischen Gasphasenoxidation zu Acrylsäure wurde ein Gemisch folgender Zusammensetzung (Tabelle 1) mit einer Temperatur von 270 °C erhalten:

**Tabelle 1:**

| Komponente | Konzentration, Gew.-% |
|---|---|
| Wasser | 4,4 |
| Formaldehyd | 0,2 |
| Essigsäure | 0,4 |
| Acrylsäure | 10,1 |
| Maleinsäureanhydrid | 0,07 |
| Benzoesäure | 0,02 |
| Acrolein | 0,1 |
| Phthalsäureanhydrid | 0,01 |
| Propionsäure | 0,002 |
| Maleinsäure | 0,0 |
| Allylacrylat | 0,001 |
| Benzaldehyd | 0,001 |
| Furfural | 0,001 |
| Phenothiazin | 0,0 |
| Stickstoff | Rest (76,545) |
| Sauerstoff | 3,6 |
| Kohlenoxid | 0,75 |
| Kohlendioxid | 2,6 |
| Propen | 0,5 |
| Propan | 0,7 |

Das Gemisch (3040 g/h) wurde von unten in eine Kolonne eingeführt, die schematisch im wesentlichen wie die in der Figur gezeigte Kolonne ausgebildet ist. Es wurde eine Kolonne mit Glockenböden verwendet. Die Kolonne war 2,6 m hoch und hatte einen Durchmesser von 8 cm. Die Anzahl der Böden betrug 27. Die Temperatur im Kolonnensumpf betrug 120 °C. Die Kondensationswärme wurde über Wärmeübertrager an den Böden 1 und 27 abgeführt. Am Kopf der Kolonne wurde kontinuierlich Phenothiazin als Stabilisator zugegeben.

Vom Kolonnensumpf wurde eine Schwersiederfraktion der in Tabelle 2 angegebenen Zusammensetzung von 1 g/h bei 120 °C ausgeschleust:

**Tabelle 2**

| Komponente | Konzentration, Gew.-% |
|---|---|
| Wasser | 0,6 |
| Formaldehyd | 0,002 |
| Essigsäure | 0,403 |
| Acrylsäure | 40 |
| Maleinsäureanhydrid | 0,9 |
| Benzoesäure | 9,0 |
| Acrolein | 0,006 |
| Phthalsäureanhydrid | 3,6 |
| Propionsäure | 0,008 |
| Maleinsäure | 0 |
| Allylacrylat | 0,002 |
| Benzaldehyd | 0,006 |
| Furfural | 0,009 |
| Phenothiazin | Rest (45,464) |
| Stickstoff | 0 |
| Sauerstoff | 0 |
| Kohlenoxid | 0 |
| Kohlendioxid | 0 |
| Propen | 0 |
| Propan | 0 |

Am Boden 3 wurde über Seitenabzug folgende Mittelsiederfraktion (Tabelle 3) von 350 g/h bei 93 °C aus der Kolonne abgezogen:

**Tabelle 3**

| Komponente | Konzentration, Gew.-% |
|---|---|
| Wasser | 1,1 |
| Formaldehyd | 0,004 |
| Essigsäure | 1,0 |
| Acrylsäure | Rest (96,914) |
| Maleinsäureanhydrid | 0,6 |
| Benzoesäure | 0,2 |
| Acrolein | 0,008 |
| Phthalsäureanhydrid | 0,1 |
| Propionsäure | 0,02 |
| Maleinsäure | 0 |
| Allylacrylat | 0,01 |
| Benzaldehyd | 0,004 |
| Furfural | 0,01 |
| Phenothiazin | 0,03 |
| Stickstoff | 0 |
| Sauerstoff | 0 |
| Kohlenoxid | 0 |
| Kohlendioxid | 0 |
| Propen | 0 |
| Propan | 0 |

Am Boden 27 wurde folgende Leichtsiederfraktion (Tabelle 4) von 90 g/h bei 34 °C abgenommen:

**Tabelle 4**

| Komponente | Konzentration, Gew.-% |
|---|---|
| Wasser | Rest (87,69) |
| Formaldehyd | 0,08 |
| Essigsäure | 8,2 |
| Acrylsäure | 4,0 |
| Maleinsäureanhydrid | 0 |
| Benzoesäure | 0 |
| Acrolein | 0,03 |
| Phthalsäureanhydrid | 0 |
| Propionsäure | 0 |
| Maleinsäure | 0 |
| Allylacrylat | 0 |
| Benzaldehyd | 0 |
| Furfural | 0 |
| Phenothiazin | 0 |
| Stickstoff | 0 |
| Sauerstoff | 0 |
| Kohlenoxid | 0 |
| Kohlendioxid | 0 |
| Propen | 0 |
| Propan | 0 |

Die nicht kondensierbaren Komponenten (Abgas) wurden über Kopf abgeführt. Ihre Zusammensetzung war bei 2640 g/h und 25 °C wie folgt:

**Tabelle 5**

| Komponente | Konzentration, Gew.-% |
|---|---|
| Wasser | 2,0 |
| Formaldehyd | 0,2 |
| Essigsäure | 0,09 |
| Acrylsäure | 0,03 |
| Maleinsäureanhydrid | 0 |
| Benzoesäure | 0 |
| Acrolein | 0,1 |
| Phthalsäureanhydrid | 0 |
| Propionsäure | 0 |
| Maleinsäure | 0 |
| Allylacrylat | 0 |
| Benzaldehyd | 0 |
| Furfural | 0 |
| Phenothiazin | 0 |
| Stickstoff | Rest (88,18) |
| Sauerstoff | 4,1 |
| Kohlenoxid | 0,9 |
| Kohlendioxid | 3,0 |
| Propen | 0,6 |
| Propan | 0,8 |

Wie aus dem Vergleich von Tabelle 3 mit den übrigen Tabellen ersichtlich ist, wird durch den Einsatz einer Kolonne zur Kondensation Acrylsäure hoher Reinheit sowie eine gute Auftrennung der weiteren Komponenten erhalten.

## Patentansprüche

1. Verfahren zur fraktionierten Kondensation eines Gasgemisches, das neben Acrylsäure oder Methacrylsäure noch mindestens eine weitere kondensierbare Komponente und außerdem einen hohen Anteil einer oder mehrerer nicht kondensierbarer Komponenten enthält, **dadurch gekennzeichnet, daß** man das Gasgemisch durch eine Kolonne mit trennwirksamen Einbauten führt und die kondensierbaren Komponenten durch Kühlung auskondensiert.

2. Verfahren nach Anspruch 1, **dadurch gekennzeichnet, daß** das Gasgemisch als Rohprodukt der katalytischen Gasphasenoxidation von C₃-/C₄-Alkanen, -Alkenen, -Alkanolen und/oder -Alkanalen und/oder Vorstufen davon zu Acrylsäure oder Methacrylsäure vorliegt.

3. Verfahren nach Anspruch 1 oder 2, **dadurch gekennzeichnet, daß** das heiße Gasgemisch vor der Auskondensation abgekühlt wird.

4. Verfahren nach einem der Ansprüche 1 bis 3, **dadurch gekennzeichnet, daß** ein heißes Gasgemisch mit einer Schwersieder-, Mittelsieder- und Leichtsiederfraktion kondensiert wird.

5. Verfahren nach Anspruch 4, **dadurch gekennzeichnet, daß** jede der Schwersieder-, Mittelsieder- und Leichtsiederfraktionen eine oder mehrere Komponenten enthält.

6. Verfahren nach einem der Ansprüche 1 bis 5, **dadurch gekennzeichnet, daß** ein heißes Gasgemisch mit zwischen 20 und 100 Gew.-% nicht kondensierbaren Komponenten bezogen auf 100 Gew.-% Gasgemisch kondensiert wird.

7. Verfahren nach einem der Ansprüche 1 bis 6, **dadurch gekennzeichnet, daß** ein heißes Gasgemisch mit einer Temperatur zwischen 100 und 350 °C kondensiert wird.

8. Verfahren nach einem der Ansprüche 1 bis 7, **dadurch gekennzeichnet, daß** eine Kolonne mit einer oder mehreren Kühlvorrichtungen verwendet wird.

9. Verfahren nach einem der Ansprüche 1 bis 8, **dadurch gekennzeichnet, daß** als trennwirksame Kolonneneinbauten Packungen, Füllkörper und/oder Böden verwendet werden.

10. Verfahren zur Herstellung von Acrylsäure oder Methacrylsäure durch katalytische Gasphasenoxidation von C₃-/C₄-Alkanen, -Alkenen, -Alkanolen und/oder -Alkanalen und/oder Vorstufen davon unter Entstehung eines Rohproduktes, das neben Acrylsäure oder Methacrylsäure noch mindestens ein Nebenprodukt und/oder mindestens einen nichtumgesetzten Ausgangsstoff enthält, **dadurch gekennzeichnet, daß** das gasförmige Rohprodukt nach dem Verfahren gemäß Anspruch 1 kondensiert wird.

## Claims

1. A process for the fractional condensation of a gas mixture which, in addition to acrylic acid or methacrylic acid, also contains at least one further condensable component and additionally a high proportion of one or more noncondensable components, wherein the gas mixture is passed through a column having separatory internals and the condensable components are condensed by cooling.

2. A process as claimed in claim 1, wherein the gas mixture is present as a crude product of the catalytic gas-phase oxidation of C₃-/C₄-alkanes, - alkenes, -alkanols and/or -alkanals and/or intermediates thereof to give acrylic acid or methacrylic acid.

3. A process as claimed in claim 1 or 2, wherein the hot gas mixture is cooled before the condensation.

4. A process as claimed in any of claims 1 to 3, wherein a hot gas mixture having a high boiler fraction, medium boiler fraction and low boiler fraction is condensed.

5. A process as claimed in claim 4, wherein each of the high boiler, medium boiler and low boiler fractions contains one or more components.

6. A process as claimed in any of claims 1 to 5, wherein a hot gas mixture comprising from 20 to 100% by weight of noncondensable components, based on 100% by weight of gas mixture, is condensed.

7. A process as claimed in any of claims 1 to 6, wherein a hot gas mixture at from 100 to 350°C is condensed.

8. A process as claimed in any of claims 1 to 7, wherein a column having one or more cooling apparatuses is used.

9. A process as claimed in any of claims 1 to 8, wherein the separatory column internals used are stacked packings, dumped packings and/or trays.

10. A process for the preparation of acrylic acid or methacrylic acid by catalytic gas-phase oxidation of C₃-/C₄-alkanes, -alkenes, -alkanols and/or -alkanals and/or intermediates thereof with formation of a crude product which, in addition to acrylic acid or methacrylic acid, also contains at least one byproduct and/or at least one unconverted starting material, wherein the gaseous crude product is condensed by a process as claimed in claim 1.

## Revendications

1. Procédé de condensation fractionnée d'un mélange gazeux, qui, outre de l'acide acrylique ou de l'acide méthacrylique, contient encore au moins un autre composant condensable et en outre une fraction élevée d'un ou de plusieurs composants non condensables, **caractérisé en ce qu'**on conduit le mélange gazeux au travers d'une colonne garnie d'éléments encastrés ayant une action de séparation et **en ce qu'**on condense les composants condensables par refroidissement.

2. Procédé suivant la revendication 1, **caractérisé en ce que** le mélange gazeux se présente sous la forme d'un produit brut de l'oxydation catalytique en phase gazeuse d'alcanes, alcènes, alcanols et/ou alcanals en C₃/C₄ et/ou de leurs progéniteurs en acide acrylique ou méthacrylique.

3. Procédé suivant l'une des revendications 1 et 2, **caractérisé en ce que** le mélange gazeux chaud est refroidi avant la séparation par condensation.

4. Procédé suivant l'une des revendications 1 à 3, **caractérisé en ce qu'**un mélange gazeux chaud comportant une fraction de substances à haut point d'ébullition, à point d'ébullition moyen et à bas point d'ébullition est condensé.

5. Procédé suivant la revendication 4, **caractérisé en ce que** chacune des fractions à haut point d'ébullition, point d'ébullition moyen et bas point d'ébullition contient un ou plusieurs composants.

6. Procédé suivant l'une des revendications 1 à 5, **caractérisé en ce qu'**un mélange gazeux chaud comportant entre 20 et 100% en poids de composants non condensables par rapport à 100% en poids du mélange gazeux est condensé.

7. Procédé suivant l'une des revendications 1 à 6, **caractérisé en ce qu'**un mélange gazeux chaud ayant une température comprise entre 100 et 350°C est condensé.

8. Procédé suivant l'une des revendications 1 à 7, **caractérisé en ce qu'**on utilise une colonne comportant un ou plusieurs dispositifs de refroidissement.

9. Procédé suivant l'une des revendications 1 à 8, **caractérisé en ce que**, comme éléments encastrés dans la colonne, à action de séparation, on utilise des garnissages, des corps de remplissage et/ou des plateaux.

10. Procédé de préparation d'acide acrylique ou d'acide méthacrylique par oxydation catalytique en phase gazeuse d'alcanes, alcènes, alcanols et/ou alcanals en C₃/C₄ et/ou de leurs progéniteurs avec formation d'un produit brut qui, outre de l'acide acrylique ou de l'acide méthacrylique, contient encore au moins un produit secondaire et/ou au moins une matière de départ n'ayant pas réagi, **caractérisé en ce que** le produit brut gazeux est condensé selon le procédé suivant la revendication 1.
